# EUROPÄISCHE PATENTSCHRIFT

(11) **EP 2 448 538 B1**
(45) Veröffentlichungstag und Bekanntmachung des Hinweises auf die Patenterteilung: **15.05.2013**
(21) Anmeldenummer: 10739847.1
(22) Anmeldetag: 28.06.2010
(51) Int. Cl.: A61F 13/20

(54) **VERFAHREN FÜR EINE ABSCHLIESSENDE FORMGEBUNG EINES TAMPONS**
PROCESS FOR FINAL FORMING OF A TAMPON
PROCEDE DE MISE EN FORME FINALE D'UN TAMPON

(30) Priorität: 29.06.2009 AT 10092009
(43) Veröffentlichungstag der Anmeldung: 09.05.2012
(73) Patentinhaber: Ruggli Projects AG, 6332 Hagendorn (CH)
(72) Erfinder: ROLLI, Kilian, CH-5400 Baden (CH)
(74) Vertreter: Burger, Hannes
(86) Internationale Anmeldenummer: PCT/EP2010/003842
(87) Internationale Veröffentlichungsnummer: WO 2011/000508

(56) Entgegenhaltungen:
- EP-A2- 1 304 095
- WO-A1-2004/100847
- DE-A1- 1 815 541
- DE-A1-102005 037 065

## Beschreibung

Die Erfindung betrifft ein Verfahren für eine einen Herstellungsprozess abschließende Formgebung eines Tampons gemäß dem Oberbegriff des Anspruchs 1.

Weiters betrifft die Erfindung eine Vorrichtung für eine einen Herstellungsprozess abschließende Formgebung eines Tampons, gemäß dem Oberbegriff des Anspruchs 8.

Tampons sind allgemein bekannte Hygieneartikel, welche aus Wattebändern durch Rollen und anschließendem Verdichten in einer Pressvorrichtung hergestellt werden können, wobei sich üblicherweise eine herstellungsbedingt kreiszylinderförmige Grundform des Tampons ergibt. Oftmals ist es jedoch gewünscht, Tampons herzustellen welche zusätzlich noch eine spezielle Formgebung, beispielsweise in Form abschnittsweise unterschiedlicher Querschnitte aufweisen. Ein derartiger Tampon ist beispielsweise aus der DE 212004000071 U1 bekannt geworden. Um Beschädigungen der Faserstruktur des Tampons zu vermeiden, sind bei der Herstellung eines solchen Tampons jedoch relativ aufwendige Verfahren notwendig.
Aus der WO 2004/100847 A1 ist ein weiteres Verfahren zur Formgebung eines Tampons bekannt gweorden. Bei dem bekannten Verfahren wird ein Tamponvorformling in eine zweiteilige Form eingelegt, deren Kontur der fertige Tampon annehmen soll. Durch Zufuhr von Wärme wird erreicht, dass die Gestalt des Tampons nach Entfernen der Form stabil bleibt.

Der Erfindung liegt daher die Aufgabe zugrunde, Tampons mit unterschiedlichen Querschnitten herzustellen, ohne die Faserstruktur zu verletzen.

Diese Aufgabe wird mit einem Verfahren der eingangs genannten Art erfindungsgemäß durch die kennzeichnenden Merkmale des Anspruchs 1 gelöst.

Durch eine eventuelle Erwärmung des Tampons vor bzw. während der Verdichtung kann auf einfache Weise eine formstabile bzw. dauerhafte Umformung des Tampons erleichtert werden. Unter dem Begriff "abschließende Formgebung" wird in diesem Dokument verstanden, dass es sich hierbei um den letzten Formgebungsprozess des Herstellungsverfahrens handelt. Das erfindungsgemäße Verfahren kann einem herkömmlichen Herstellungsverfahren nachgeschaltet werden, welches zur Erzeugung eines Tampons verwendet wird. Da als Ausgangsprodukt für das erfindungsgemäße Verfahren vorteilhafterweise ein nach einem herkömmlichen Herstellungsverfahren bereits "fertiggestellter" Tampon verwendet werden kann, lässt es sich auf einfache Weise mit beliebigen Herstellungsmethoden für Tampons kombinieren und kann in bereits bestehende Produktionsprozesse integriert werden.

Unter dem Begriff "Rillenebene" wird in diesem Dokument eine Ebene verstanden, in welcher eine in Längsrichtung der Rille umlaufende und die Form des Rillenumfangs beschreibende, zweidimensionale Kurve liegt. Diese Kurve kann beispielsweise ein Kreis, eine Ellipse oder von polygonaler oder beliebiger anderer Form sein.

Gemäß der Erfindung ist es hierbei vorgesehen, dass eine Mantelfläche des Tampons abschnittsweise von zumindest einer ersten Pressform mit gegenläufig zueinander bewegbaren, je konkav ausgebildeten und über ihren Verlauf weniger stark als die Mantelfläche des Tampons gekrümmten Pressabschnitten, die so angeordnet sind, dass sich durch eine Pressung des Tampons mittels der Pressabschnitte eine konvexe Oberffächenform eines gepressten Bereichs des Tampons ergibt, umfasst und durch eine Bewegung der Pressabschnitte aufeinander zu verdichtet wird, wobei ein verdichteter Bereich nach Entfernen der Pressabschnitte im wesentlichen eine mit dem Konturverlauf der Pressabschnitte korrespondierende Kontur aufweist.

Hierbei ist es günstig, wenn der von den Pressabschnitten in einem geschlossenen Zustand der Pressform begrenzte Bereich von größerer Länge und geringerer Breite als ein Durchmesser des zu verdichtenden Tampons vor seinem Verdichten ist, Auf die soeben genannte Weise lässt sich auf einfache Weise eine Verdichtung des Tampons bewirken, wobei durch entsprechende Wahl der Krümmungsradien der Pressabschnitte bzw. durch geeignete Krümmung der Pressabschnitte gewährleistet wird, dass es zu keiner Verletzung der Oberfläche bzw. zu keiner Durchtrennung von Fasern des Tampons kommt.

Gemäß einer günstigen Weiterentwicklung der Erfindung ist es vorgesehen, dass ein zuvor mittels der ersten Pressform verdichteter Bereich des Tampons mit zumindest einer zweiten Pressform mit gegenläufig zueinander bewegbaren, jeweils konkav ausgebildeten Pressabschnitten, die so angeordnet sind, dass sich durch eine Pressung des Tampons mittels der Pressabschnitte eine konvexe Oberflächenform des gepressten Bereichs des Tampons ergibt, umfasst und umgeformt wird, wobei die Pressabschnitte der zweiten Pressform stärker gekrümmt sind als die Pressabschnitte der zuvor verwendeten ersten Pressform jedoch weniger stark gekrümmt sind als ihnen zugewandte Abschnitte des zuvor verdichteten Bereichs des Tampons. Durch die aufeinander folgende Pressung des Tampons mit Pressformen unterschiedlicher Krümmungen bzw. Größen lässt sich auf einfache Weise eine gewünschte Endform erzielen. Durch die erste Verdichtung und Umformung kann der Tampon in eine Form gebracht werden, welche dann mit der zweiten, kleineren Pressform bearbeitet werden kann, ohne die Oberfläche des Tampons bzw. dessen Fasern zu verletzen.

Umlaufende Verdichtungen beliebigen Verlaufs lassen sich dadurch herstellen, dass in die Mantelfläche des Tampons durch Verdichten des Tampons mit der ersten Pressform eine gemäß einer ersten Kurvenform umlaufende Rille und durch Verdichten des Tampons mit der zweiten Pressform eine weitere gemäß einer zweiten Kurvenform umlaufende Rille eingeprägt wird. In diesem Zusammenhang hat es sich als günstig herausgestellt, dass die gemäß der ersten Kurvenform umlaufende Rille und/oder die gemäß der zweiten Kurvenform umlaufende Rille in sich in Umfangsrichtung der Mantelfläche geschlossen ist.

Um eine kreisförmig Verdichtung mit einem geringeren Durchmesser als dem Durchmesser des Tampons herstellen zu können, kann in einem ersten Schritt mit der Pressform eine erste in Umfangsrichtung oval um die Mantelfläche umlaufende Rille eingeprägt werden, wobei in einem weiteren Schritt die oval umlaufende Rille durch Pressen mit der weiteren Pressform auf eine im wesentlichen kreisförmig umlaufende Rille umgeformt wird.

Eine bevorzugte Variante der Erfindung besteht darin, dass der Tampon in einem bereits in einer Schutzhülle verpackten Zustand verdichtet und umgeformt wird. Durch diese Ausführungsform der Erfindung werden auf einfache Weise Probleme bei der Verpackung eines komplex geformten Tampons vermieden, da ja die Formgebung erst nach der Verpackung des Tampons erfolgt.

Die oben genannte Aufgabe kann auch mit einer Vorrichtung der eingangs genannten Art gelöst werden, welche gemäß dem kennzeichnenden Teil des Anspruchs 8 ausgebildet ist.

Gemäß der Erfindung weist die Vorrichtung zumindest eine Pressform mit im wesentlichen gegenläufig zueinander bewegbaren, jeweils konkav ausgebildeten Pressabschnitten auf, wobei die Pressabschnitte in einem geschlossenem Zustand der Pressform einen Pressbereich begrenzen und so angeordnet sind, dass sich durch eine Pressung des Tampons mittels der Pressabschnitte eine konvexe Oberflächenform des gepressten Bereichs des Tampons ergibt.

Die Einschubrichtung des Tampons in die Pressform kann im wesentlichen parallel zu einer Pressebene verlaufen, wobei jeder Pressabschnitt entsprechend der zu erzielenden Formgebung des zu verdichteten Bereichs geformt sein kann.

Um die erforderliche Temperatur während des Umformungsvorganges in den Tampon einbringen zu können, kann zumindest eine Pressform beheizbar sein. Alternativ hierzu könnte jedoch auch der Tampon selbst vorgeheizt werden.

Gemäß einer Variante der Erfindung, welche sich unter anderem durch einen einfachen Aufbau auszeichnet, kann die Pressform zumindest zwei plattenförmige Pressbacken aufweisen, wobei einander zugewandte Abschnitte der Stirnkanten der Pressbacken als Pressabschnitte vorgesehen sind.

Darüber hinaus kann die Vorrichtung zumindest zwei Pressformen aufweisen, die je wie oben beschrieben ausgebildet sein können, wobei die Pressabschnitte der zweiten Pressform stärker gekrümmt sind als die Pressabschnitte der ersten Pressform. Mit einer derart ausgebildeten Vorrichtung lassen sich auf einfache Weise Verdichtungen mit kleineren Durchmessern als der Tampondurchmesser herstellen, da durch ein Vorpressen des Tampons mit der ersten Pressform der Radius des verdichteten Bereichs so verringert werden kann, dass er in die zweite kleiner Pressform passt. Das Verfahren ließe sich noch beliebig so lange fortsetzen, solange es zu keiner Schädigung der Fasern des Tampons kommt.

Die Erfindung samt weiteren Vorteilen ist im Folgenden anhand eines nicht einschränkenden, in den Zeichnungen dargestellten Ausführungsbeispiels näher erläutert. In diesen zeigen schematisch:
- Fig. 1: eine Pressform einer erfindungsgemäßen Vorrichtung mit geöffneten Pressbacken und einem zwischen diesen befindlichen Tampon;
- Fig. 2: die Pressform aus Fig. 1 mit geschlossenen Pressbacken;
- Fig. 3: eine perspektivische Ansicht eines Tampons vor einem Umformungsprozess mit der Pressform aus Fig. 1 und 2;
- Fig. 4: einen Längsschnitt durch den Tampon aus Fig. 3;
- Fig. 5: eine perspektivische Ansicht auf den Tampon aus Fig. 3 nach einem Umformungsprozess durch Pressen mit der Pressvorrichtung aus Fig. 1 und 2;
- Fig. 6: einen Längsschnitt durch den Tampon aus Fig. 5;
- Fig. 7: eine Draufsicht auf einen teilweisen Querschnitt auf die Darstellung aus Fig. 1;
- Fig. 8: eine Draufsicht auf einen teilweisen Querschnitt auf die Darstellung aus Fig. 2;
- Fig. 9: eine perspektivische Ansicht auf eine weitere Pressform der erfindungsgemäßen Vorrichtung mit geöffneten Pressbacken, wobei die Pressabschnitte der Pressbacken einen geringeren Krümmungsradius aufweisen als die Pressabschnitte der Pressbacken der in Fig. 1, 2, 7 und 8 dargestellten Pressform;
- Fig. 10: die Pressform aus Fig. 9 mit geschlossenen Pressbacken;
- Fig. 11: eine perspektivische Ansicht eines Tampons vor dem Pressen mit der Pressform aus Fig. 9 und 10;
- Fig. 12: einen Längsschnitt durch den Tampon aus Fig. 11;
- Fig. 13: eine perspektivische Ansicht des Tampons aus Fig. 11 nach dem Pressen mit der Pressvorrichtung aus Fig. 9 und 10;
- Fig. 14: einen Längsschnitt durch den Tampon aus Fig. 13;
- Fig. 15: eine Draufsicht auf einen teilweisen Querschnitt der Darstellung aus Fig. 9 und
- Fig. 16: eine Draufsicht auf einen teilweisen Querschnitt der Darstellung aus Fig. 10.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

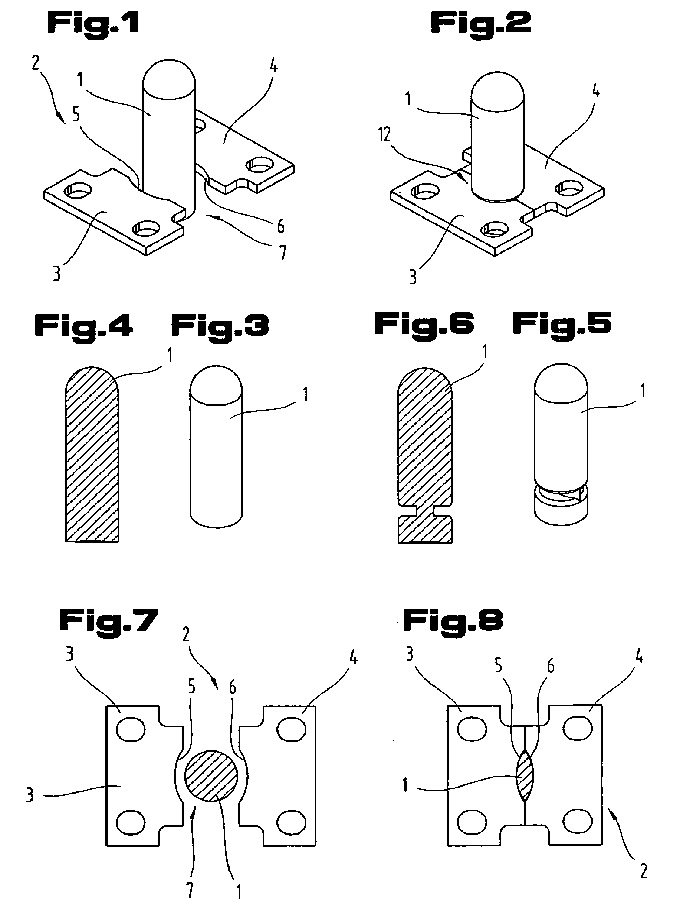

Fig. 1 zeigt eine erfindungsgemäße Vorrichtung. Die Vorrichtung ist dazu eingerichtet, einen im Allgemeinen zylindrischen Tampon 1 in radialer Richtung zumindest abschnittsweise unter thermischen Einfluss durch Druck zu verdichten. "Im Allgemeinen zylinderförmig" bezieht sich hierbei auf die übliche Form von Tampons, wie sie aus dem Stand der Technik gut bekannt ist, umfasst aber darüber hinaus auch abgeflachte oder teilweise abgeflachte Zylinder, gebogene Zylinder und Formen, welche verschiedene Querschnittbereiche aufweisen.

Gemäß Fig. 1 kann die erfindungsgemäße Vorrichtung zur Umformung eines Tampons 1 eine erste Pressform 2 mit gegenläufig zueinander bewegbaren Pressbacken 3 und 4 aufweisen. Die Pressbacken 3 und 4 weisen konkave Pressabschnitte 5 und 6 auf, die in Bezug auf eine normal zu einer Bewegungsrichtung der Pressabschnitte verlaufenden, geometrischen Mittelachse eines zwischen den Pressabschnitten 5 und 6 liegenden Pressbereiches 7 bzw. zueinander so angeordnet sind, dass durch Pressen eines Oberflächenabschnitts des Tampons 1 mittels der Pressabschnitte 5 und 6 eine konvexe Form des gepressten Oberflächenabschnitts erhalten wird. In einem zusammengepressten Zustand ist der Pressbereich 7 von dem in ihm befindlichen Abschnitts des Tampons 1 ausgefüllt.

Um ein Pressen des Tampons 1 ohne ein Herausquellen von Watte in eine Bewegungsebene der Pressabschnitte 5 und 6 zu ermöglichen und somit eine Verletzung der Oberfläche des Tampons zu verhindern, sind die Pressabschnitte 5 und 6 weniger stark gekrümmt als die Mantelfläche des Tampons 1. Durch eine Bewegung der Pressabschnitte 5 und 6 aufeinander zu kann der Tampon 1 in dem von den Pressbacken 3 und 4 umfassten Bereich verdichtet werden, wobei ein verdichteter Bereich nach Entfernen der Pressabschnitte im wesentlichen eine mit dem Konturverlauf der Pressabschnitte 5 und 6 korrespondierende Kontur aufweist. Jeder Pressabschnitt 5 und 6 ist daher entsprechend der zu erzielenden Formgebung des zu verdichteten Bereichs des Tampons 1 geformt. Wie aus Fig. 1 weiters ersichtlich ist, können die Pressbacken 3 und 4 plattenförmig ausgebildet sein, wobei einander zugewandte Abschnitte der Stirnkanten der Pressbacken 3 und 4 als Pressabschnitte 5 und 6 vorgesehen sind. Alternativ zur Verwendung von nur zwei Pressbacken 3 und 4 können auch mehrere Pressbacken, welche vorzugsweise radial um den Pressbereich 7 angeordnet sind, vorgesehen sein.

Die Einschubrichtung des Tampons 1 in die Pressform 2 bzw. in den Pressbereich 7 ist im wesentlichen parallel zur geometrischen Mittelachse des Pressbereichs 7, d.h. parallel zur Pressebene. Die Pressbacken 3 und 4 können auf hier nicht dargestellten Schlitten montiert sein, die aufeinander zu und voneinander weg bewegt werden können, um ein Öffnen und Pressen zu ermöglichen. Zum Einführen des Tampons 1 kann eine ebenfalls nicht dargestellte hülsenförmige Führung des Tampons 1 vorgesehen sein.

Weiters kann die Pressform 2 beheizbar sein, beispielsweise mittels in Bohrungen bzw. Ausnehmungen der Pressbacken 3 und 4 eingeführter Heizpatronen.

Um Abschnitte der Mantelfläche des Tampons 1 zu verdichten, können die Pressbacken 3 und 4 aus der in Fig. 1 dargestellten Position in die in Fig. 2 dargestellte Position gebracht werden. Erfindungsgemäß wird hierbei der Tampon 1 von den Pressabschnitten 5 und 6 umfasst und das Material des Tampons 1 in einem umfassten Bereich, bevorzugterweise unter thermischem Einfluss durch eine Bewegung der Pressabschnitte 5 und 6 aufeinander zu verdichtet. Die Verdichtung des Tampons 1 mittels der Pressbacken 3 und 4 erfolgt bevorzugterweise im wesentlichen normal zur Längsachse des Tampons 1, die im wesentlichen parallel zur Pressebene verläuft. Die Längsachse bezieht sich hierbei auf die längste lineare Abmessung des Tampons. Der Querschnitt bezieht sich auf eine Scheibe, die rechtwinklig zur Längsachse entnommen ist. Nach dem Verdichten weist der umfasste und umgeformte Bereich des Tampons 1 eine den Pressabschnitten 5 und 6 entsprechende Kontur sowie einen geringeren Querschnitt als an diesen Bereich anschließende Bereiche des Tampons 1 auf.

Gemäß Fig. 3 und 4 kann der Tampon 1, welcher aus einem saugfähigen Material hergestellt ist, vor dem Umformungsprozess eine im wesentlichen einem Kreiszylinder entsprechende Mantelfläche aufweisen. Besonders vorteilhaft ist es, wenn der Tampon 1 vor dem Umformungsprozess bereits in einer Schutzhülle verpackt ist.

Wie aus Fig. 5 und 6 ersichtlich ist der Tampon 1 nach einem Pressen, welches bevorzugterweise unter Wärmeeinfluss erfolgt, an der verdichteten Stelle umgeformt und weist dort einen geringeren Querschnittsdurchmesser als die daran anschließenden Bereiche des Tampons 1 auf.

Wie aus Fig. 7 und 8 zu erkennen ist, kann der von den Pressabschnitten in einem geschlossenen Zustand der Pressform 2 begrenzte Bereich von größerer Länge und geringerer Breite als ein Durchmesser des zu verdichtenden Tampons 1 vor seinem Verdichten sein.

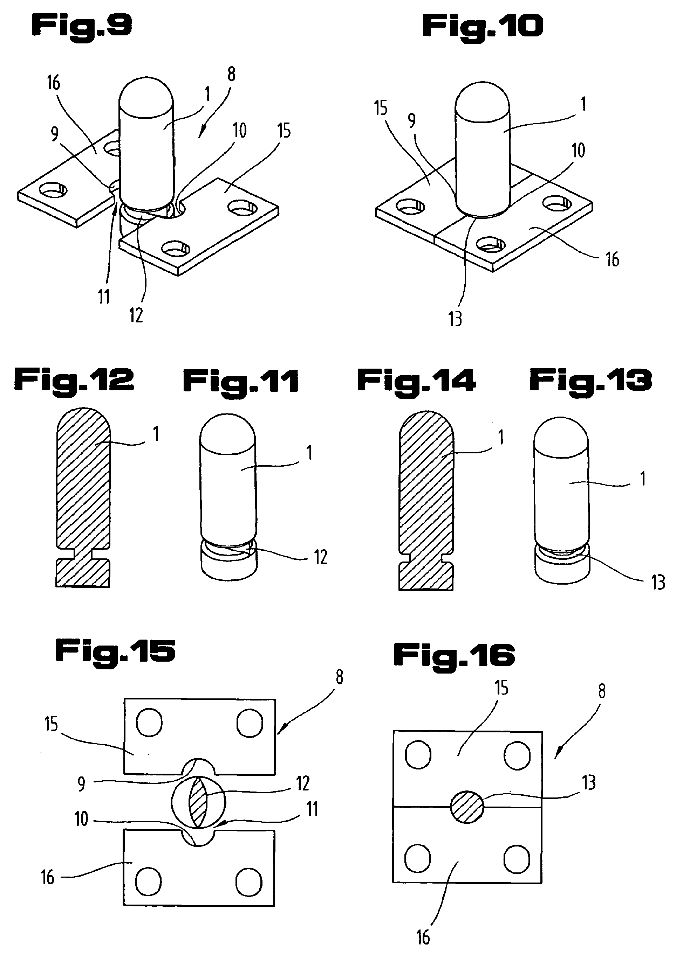

Wie aus Fig. 9 ersichtlich, kann die Vorrichtung eine weitere Pressform 8 mit gegenläufig in einer Ebene zueinander bewegbaren Pressabschnitten 9 und 10 aufweisen. Die Pressabschnitte 9 und 10 der weiteren Pressform 8 können so wie bei der in den Figuren 1, 2, 7 und 8 dargestellten und oben beschriebene Pressform 2 in Bezug auf eine normal zu einer Bewegungsrichtung der Pressabschnitte 9 und 10 verlaufenden, geometrischen Mittelachse eines zwischen den Pressabschnitten 9 und 10 liegenden Pressbereiches 11 bzw. zueinander so angeordnet sein, dass sich durch Pressen eines Oberflächenabschnitts des Tampons 1 eine konvexe Oberfläche des gepressten Bereichs ergibt.

Die Pressabschnitte 5 und 6 der ersten Pressform 2 sind jedoch vorteilhafterweise weniger stark gekrümmt als die Pressabschnitte 9 und 10 der zweiten Pressform 8. Die der zweiten Pressform 8 zugewandte Abschnitte des zuvor verdichteten Bereichs des Tampons 1 sind hierbei stärker gekrümmt als die Pressabschnitte 5, 6 der ersten Pressform 2. Auf diese Weise lässt sich gewährleisten, dass es während des Pressens zu keinem Herausquellen der Watte in eine Bewegungsebene der Pressabschnitte 9 und 10 kommt.

Auch die zweite Pressform 8 kann so wie die erste beheizbar sein. An dieser Stelle sei erwähnt, dass sich die Pressformen 2 und 8 lediglich durch die Form bzw. die Krümmung ihrer Pressabschnitte 5 und 6 bzw. 9 und 10 unterscheiden. Die zweite Pressform 8 kann ebenfalls plattenförmige Pressbacken 15 und 16 aufweisen, wobei die Stirnseiten durch die Pressabschnitte 9 und 10 gebildet sind.

Entsprechend dem erfindungsgemäßen Verfahren wird ein zuvor mittels der ersten Pressform 2 verdichteter Bereich des Tampons 1 mit der zweiten Pressform 8, bevorzugterweise ebenfalls unter thermischen Einfluss, d.h. unter eventueller Zufuhr von Wärme, umgeformt. Alternativ zu einer Wärmezufuhr in den Tampon 1 durch beheizbare Pressformen 2 bzw. 8 könnte auch der Tampon 1 selbst vorgeheizt werden. Als vorteilhaft hat sich beispielsweise bei Viskosefasern als Material, aus welchem die Tampons hauptsächlich hergestellt sind, eine Temperatur von ca. 15°C - 180°C in Abhängigkeit von der Produktionsgeschwindigkei herausgestellt. Auch die zweite Pressform 8 kann beheizt sein, wobei der mit der zweiten Pressform durchgeführte Verdichtungsvorgang bevorzugterweise bei gleicher Temperatur wie der erste Verdichtungsvorgang erfolgen kann. Der mit der zweiten Pressform 8 durchgeführte Verdichtungsvorgang kann aber auch bei einer geringeren oder höheren Temperatur als der mit der ersten Pressform 2 vorgenommene Verdichtungsvorgang erfolgen.

An dieser Stelle sei jedoch ausdrücklich angemerkt, dass das erfindungsgemäße Verfahren auch ohne thermischen Einfluss, d.h. ohne Erwärmung des Tampons 1 oder der Pressformen 2 bzw. 8, durchgeführt werden kann. Die Verdichtung des Tampons 1 unter Wärmeeinfluss stellt lediglich eine bevorzugte Variante der Erfindung dar.

Durch Verdichten des Tampons 1 mit der ersten Pressform 2 kann eine gemäß einer ersten Kurvenform umlaufende Rille 12 in die Mantelfläche des Tampons 1 eingeprägt werden.

Hierauf kann durch Verdichten des Tampons 1 mit der zweiten Pressform 8 eine weitere gemäß einer zweiten Kurvenform umlaufende Rille 13 eingeprägt werden. Die Kurvenformen der Rillen 12 und 13 können in sich geschlossen sein.

Wie in den Fig. 1 bis 8 dargestellt, kann in einem ersten Schritt mit der Pressform 2 eine erste in Umfangsrichtung oval um die Mantelfläche umlaufende Rille 12 eingeprägt werden. Gemäß den Darstellungen in den Figuren 9 bis 16 kann hierauf in einem weiteren Schritt die oval umlaufende Rille 12 durch Pressen mit der weiteren Pressform 8 auf eine im wesentlichen kreisförmig umlaufende Rille 13 umgeformt werden. Natürlich ist die erfindungsgemäße Lösung nicht auf oval und kreisförmig umlaufende Rillen beschränkt, sondern es sind beliebige Umfangsformen der Rillen 12 und 13 möglich. Von Bedeutung ist hierbei lediglich, dass der Tampon 1 nach der Umformung mit der ersten Pressform 2 an dem umgeformten Bereich so verdichtet ist, dass er von der zweiten Pressform 8 umfasst werden kann, ohne dass es zu einer Durchdringung des Tampons 1 kommt.

Auch ist die Erfindung nicht auf eine Verdichtung mittels der dargestellten Pressformen 2 und 8 beschränkt, so könnte die Rille 13 beispielsweise auch mittels einer oder mehrerer Rollen hergestellt werden.

Die oben beschriebenen Ausführungsbeispiele beziehen sich auf mögliche Ausführungsvarianten einer erfindungsgemäßen Vorrichtung bzw. eines erfindungsgemäßen Verfahrens, wobei an dieser Stelle bemerkt sei, dass die Erfindung nicht auf die speziell dargestellten und beschriebenen Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvarianten möglich sind, vom Schutzumfang mit umfasst.

## Patentansprüche

1. Verfahren für eine einen Herstellungsprozess abschließend Formgebung eines Tampons (1), welcher aus einem saugfähigen Material hergestellt ist, wobei durch radiales, bevorzugterweise unter thermischem Einfluss erfolgendes Verdichten zumindest eines entlang einer Mantelfläche des Tampons (1) umlaufenden Bereichs des Tampons (1) zumindest eine Rille (12,13) in die Mantelfläche eingeprägt wird, deren Rillenebene im wesentlichen normal zur Längserstreckung des Tampons verläuft,
**dadurch gekennzeichnet, dass** eine Mantelfläche des Tampons (1) abschnittsweise von zumindest einer ersten Pressform (2) mit gegenläufig zueinander bewegbaren, je konkav ausgebildeten und über ihren Verlauf weniger stark als die Mantelfläche des Tampons gekrümmten Pressabschnitten (5, 6), die so angeordnet sind, dass sich durch eine Pressung des Tampons (1) mittels der Pressabschnitte (9, 10) eine konvexe Oberflächenform eines gepressten Bereichs des Tampons (1) ergibt, umfasst und durch eine Bewegung der Pressabschnitte (5,6) aufeinander zu verdichtet wird, wobei ein verdichteter Bereich nach Entfernen der Pressabschnitte im wesentlichen eine mit dem Konturverlauf der Pressabschnitte (5,6) korrespondierende Kontur aufweist.

2. Verfahren nach Anspruch 1, **dadurch gekennzeichnet, dass** der von den Pressabschnitten (5, 6) in einem geschlossenen Zustand der Pressform (2) begrenzte Bereich von größerer Länge und geringerer Breite als ein Durchmesser des zu verdichtenden Tampons (1) vor seinem Verdichten ist.

3. Verfahren nach Anspruch 1 oder 2, **dadurch gekennzeichnet, dass** ein zuvor mittels der ersten Pressform (2) verdichteter Bereich des Tampons (1) mit zumindest einer zweiten Pressform (8) mit gegenläufig zueinander bewegbaren, jeweils konkav ausgebildeten Pressabschnitten (9, 10), die so angeordnet sind, dass sich durch eine Pressung des Tampons (1) mittels der Pressabschnitte (9, 10) eine konvexe Oberflächenform des gepressten Bereichs des Tampons (1) ergibt, umfasst und umgeformt wird, wobei die Pressabschnitte (9, 10) der zweiten Pressform (8) stärker gekrümmt sind als die Pressabschnitte (5,6) der zuvor verwendeten ersten Pressform (2) jedoch weniger stark gekrümmt sind als ihnen zugewandte Abschnitte des zuvor verdichteten Bereichs des Tampons (1).

4. Verfahren nach Anspruch 3, **dadurch gekennzeichnet, dass** in die Mantelfläche des Tampons (1) durch Verdichten des Tampons (1) mit der ersten Pressform (2) eine gemäß einer ersten Kurvenform umlaufende Rille (12) und durch Verdichten des Tampons (1) mit der zweiten Pressform (8) eine weitere gemäß einer zweiten Kurvenform umlaufende Rille (13) eingeprägt wird.

5. Verfahren nach Anspruch 4, **dadurch gekennzeichnet, dass** die gemäß der ersten Kurvenform umlaufende Rille (12) und/oder die gemäß der zweiten Kurvenform umlaufende Rille (13) in sich in Umfangsrichtung der Mantelfläche geschlossen ist.

6. Verfahren nach Anspruch 5, **dadurch gekennzeichnet, dass** in einem ersten Schritt mit der ersten Pressform (2) eine erste in Umfangsrichtung oval um die Mantelfläche umlaufende Rille (12) eingeprägt wird, wobei in einem weiteren Schritt die umlaufende Rille (12) durch Pressen mit der zweiten Pressform auf eine im wesentlichen kreisförmig umlaufende Rille (13) umgeformt wird.

7. Verfahren nach einem der Ansprüche 1 bis 6, **dadurch gekennzeichnet, dass** der Tampon (1) in einem bereits in einer Schutzhülle verpackten Zustand verdichtet und umgeformt wird.

8. Vorrichtung für eine einen Herstellungsprozess abschließende Formgebung eines Tampons (1), welcher aus einem saugfähigen Material hergestellt ist, wobei die Vorrichtung dazu eingerichtet ist, zumindest einen entlang einer Mantelfläche des Tampons (1) umlaufenden Bereich zumindest teilweise in radialer Richtung, bevorzugterweise unter thermischem Einfluss, durch Druckausübung zu verdichten und eine Rille (12, 13) mit einer im wesentlichen normal zur Längsachse des Tampons (1) verlaufenden Rillenebene in die Mantelfläche einzuprägen,
**dadurch gekennzeichnet, dass** die Vorrichtung zumindest eine Pressform (2) mit im wesentlichen gegenläufig zueinander bewegbaren, jeweils konkav ausgebildeten Pressabschnitten (5, 6) aufweist, wobei die Pressabschnitte (9, 10) in einem geschlossenem Zustand der Pressform (2) einen Pressbereich begrenzen und so angeordnet sind, dass sich durch eine Pressung des Tampons (1) mittels der Pressabschnitte (9, 10) eine konvexe Oberflächenform des gepressten Bereichs des Tampons (1) ergibt

9. Vorrichtung nach Anspruch 8, **dadurch gekennzeichnet, dass** die Einschubrichtung des Tampons (1) in die Pressform (2) im wesentlichen parallel zur Pressebene verläuft.

10. Vorrichtung nach einem der Ansprüche 8 oder 9, **dadurch gekennzeichnet, dass** jeder Pressabschnitt (5, 6, 9, 10) entsprechend der zu erzielenden Formgebung des zu verdichteten Bereichs geformt ist.

11. Vorrichtung nach einem der Ansprüche 8 bis 10, **dadurch gekennzeichnet, dass** zumindest eine Pressform (2, 8) beheizbar ist.

12. Vorrichtung nach einem der Ansprüche 8 bis 11, **dadurch gekennzeichnet, dass** zumindest eine Pressform (2, 8) zumindest zwei plattenförmige Pressbacken (3,4,15,16) aufweist, wobei einander zugewandte Abschnitte der Stirnkanten der Pressbacken (3, 4,15, 16) als Pressabschnitte (5, 6, 9, 10) vorgesehen sind.

13. Vorrichtung nach einem der Ansprüche 8 bis 12, **dadurch gekennzeichnet, dass** sie zumindest zwei Pressformen (2, 8) aufweist, die je nach einem der Ansprüche 10 bis 14 ausgebildet sind, wobei Pressabschnitte (9,10) der zweiten Pressform (8) stärker gekrümmt sind als die Pressabschnitte (5, 6) der ersten Pressform (2).

## Claims

1. Method for a shaping step terminating a process of producing a tampon (1) made from an absorbent material, whereby at least one groove (12, 13) is embossed into the peripheral surface by radially compressing, preferably under the effect of heat, at least one region of the tampon (1) extending circumferentially around a peripheral surface of the tampon (1), the groove plane of which extends substantially normally to the longitudinal extension of the tampon,
**characterised in that** some portions of a peripheral surface of the tampon (1) are gripped by at least a first press mould (2) with pressing sections (5, 6) which are moveable in opposite directions, each being of a concave design and less pronouncedly curved in their course than the peripheral surface of the tampon, which are disposed in such a way that a convex surface shape is produced in a pressed region of the tampon (1) when the tampon (1) is pressed by means of the pressing sections (9, 10) causing compression when the pressing sections (5, 6) are moved towards one another, and a compressed region essentially has a contour corresponding to the course of the contour of the pressing sections (5, 6) when the pressing sections are removed.

2. Method as claimed in claim 1, **characterised in that** the region bounded by the pressing sections (5, 6) when the press mould (2) is in a closed state has a longer length and shorter width than a diameter of the tampon (1) to be compressed prior to its compression.

3. Method as claimed in claim 1 or 2, **characterised in that** a region of the tampon (1) previously compressed by means of the first press mould (2) is gripped and reshaped by means of at least a second press mould (8) with pressing sections (9, 10) which are moveable in opposite directions, each being of a concave design, which are disposed in such a way that a convex surface shape is produced in the pressed region of the tampon (1) by pressing of the tampon (1) by means of the pressing sections (9, 10), and the pressing sections (9, 10) of the second press mould (8) are more pronouncedly curved than the pressing sections (5, 6) of the first press mould (2) previously used, but less pronouncedly curved than the previously compressed region of the tampon (1) facing them.

4. Method as claimed in claim 3, **characterised in that** a circumferentially extending groove (12) conforming to a first curved shape is embossed into the peripheral surface of the tampon (1) by compressing the tampon (1) using the first press mould (2) and a circumferentially extending groove (13) conforming to a second curved shape is embossed by compressing the tampon (1) using the second press mould (8).

5. Method as claimed in claim 4, **characterised in that** the circumferentially extending groove (12) conforming to the first curved shape and/ or the circumferentially extending groove (13) conforming to the second curved shape is essentially closed in the circumferential direction of the peripheral surface.

6. Method as claimed in claim 5, **characterised in that**, in a first step, a first circumferentially extending groove (12) is embossed by the first press mould (2) in an oval shape in the circumferential direction around the peripheral surface, and in a further step, the circumferentially extending groove (12) is reshaped by pressing with the second press mould to produce a circumferentially extending groove (13) of an essentially circular shape.

7. Method as claimed in one of claims 1 to 6, **characterised in that** the tampon (1) is already in a packaged state, wrapped in a protective cover, when it is compressed and reshaped.

8. Apparatus for a shaping step terminating a process for producing a tampon (1) made from an absorbent material, **characterised in that** the apparatus is set up to compress at least a circumferentially extending region along a peripheral surface of the tampon (1) at least partially in the radial direction, preferably under the effect of heat, by applying pressure, and to emboss a groove (12, 13) with a groove plane extending substantially normally to the longitudinal axis of the tampon (1), **characterised in that** the apparatus comprises at least one press mould (2) with pressing sections (5, 6) which are essentially moveable in opposite directions, each of a concave design, and the pressing sections (9, 10) bound a compression region when the press mould (2) is in a closed state and are disposed in such a way that a convex surface shape of the pressed region of the tampon (1) is produced by pressing the tampon (1) by means of the pressing sections (9, 10).

9. Apparatus as claimed in claim 8, **characterised in that** the insertion direction of the tampon (1) into the press mould (2) extends essentially parallel with the press plane.

10. Apparatus as claimed in one of claims 8 or 9, **characterised in that** each pressing section (5, 6, 9, 10) is shaped according to the shaping to be obtained in the region to be compressed.

11. Apparatus as claimed in one of claims 8 to 10, **characterised in that** at least one press mould (2, 8) is heatable.

12. Apparatus as claimed in one of claims 8 to 11, **characterised in that** at least one press mould (2, 8) features at least two plate-shaped compression jaws (3, 4, 15, 16), and mutually facing sections of the edges of the compression jaws (3, 4, 15, 16) are designed as pressing sections (5, 6, 9, 10).

13. Apparatus as claimed in one of claims 8 to 12, **characterised in that** it comprises at least two press moulds (2, 8), each as claimed in one of claims 10 to 14, and the pressing sections (9, 10) of the second press mould (8) are curved more pronouncedly than the pressing sections (5, 6) of the first press mould (2).

## Revendications

1. Procédé de façonnage terminant un processus de fabrication d'un tampon (1), constitué d'un matériau absorbant, au moins une strie (12, 13), dont le plan s'étend essentiellement perpendiculairement à l'extension longitudinale du tampon, étant réalisée dans la surface externe grâce à une compression radiale, de préférence grâce à une action thermique, d'au moins une zone du tampon (1) s'enroulant le long d'une surface externe du tampon (1), **caractérisé en ce qu'**une surface externe du tampon (1) comprend au moins une première forme de compression (2) avec des sections de compression (5, 6) mobiles entre elles dans des directions opposées, de forme concave et incurvées moins fortement que la surface externe du tampon, qui sont disposées de façon à ce que, grâce à une compression du tampon (1) à l'aide des sections de compression (9, 10), une forme de surface convexe d'une zone comprimée du tampon (1) est obtenue, et est comprimée grâce à un mouvement des sections de compression (5, 6) , une zone comprimée comprenant, après retrait des sections de compression essentiellement un contour correspondant au contour des sections de compression (5, 6).

2. Procédé selon la revendication 1, **caractérisé en ce que** la zone limitée par les sections de compression (5, 6) lorsque la forme de compression (2) est fermée, présente une longueur supérieure et une largeur inférieure à un diamètre du tampon (1) à comprimer avant sa compression.

3. Procédé selon la revendication 1 ou 2, **caractérisé en ce qu'**une zone du tampon (1) préalablement comprimée par la forme de compression (2) comprend au moins une deuxième forme de compression (8) avec des sections de compression (9, 10) mobiles entre elles dans des directions opposées, de forme concave, qui sont disposées de façon à ce qu'une compression du tampon (1) à l'aide des sections de compression (9, 10) permette d'obtenir une forme de surface convexe de la zone comprimée du tampon (1), les sections de compression (9, 10) de la deuxième forme de compression (8) étant plus fortement incurvées que les sections de compression (5, 6) de la première forme de compression (2) préalablement utilisée mais moins fortement incurvées que les sections de la zone préalablement comprimée du tampon (1), orientées vers celle-ci.

4. Procédé selon la revendication 3, **caractérisé en ce que**, dans la surface externe du tampon (1), une rainure (12) épousant la première forme incurvée est réalisée par compression du tampon (1) avec la première forme de compression (2) et une autre rainure (13) épousant une deuxième forme incurvée est réalisée par compression du tampon (1) avec la deuxième forme de compression (8).

5. Procédé selon la revendication 4, **caractérisé en ce que** la rainure (12) épousant la première forme incurvée et/ou la rainure (13) épousant la deuxième forme incurvée est fermée sur la circonférence de la surface externe.

6. Procédé selon la revendication 5, **caractérisé en ce que**, dans une première étape, une première rainure (12) ovale épousant la périphérie de la surface externe est réalisée avec la première forme de compression (2), la rainure (12) périphérique étant formée, dans une étape suivante, par compression avec la deuxième forme de compression sur une rainure (13) essentiellement circulaire.

7. Procédé selon l'une des revendications 1 à 6, **caractérisé en ce que** le tampon (1) est comprimé et formé dans un état déjà compacté dans une gaine de protection.

8. Dispositif pour un façonnage terminant un processus de fabrication d'un tampon (1) constitué d'un matériau absorbant, ce dispositif étant conçu pour comprimer au moins partiellement en direction radiale, au moins une zone épousant une surface externe du tampon (1), de préférence grâce à une action thermique, par l'application d'une pression et pour réaliser une rainure (12, 13) avec un plan s'étendant essentiellement perpendiculairement à l'axe longitudinal du tampon (1),
**caractérisé en ce que** le dispositif comprend au moins une forme de compression (2) avec des sections de compression (5, 6) mobiles entre elles dans des directions opposées, de forme concave, les sections de compression (9, 10) limitant, lorsque la forme de compression (2) est fermée, une zone de compression et disposées de façon à ce que, grâce à une compression du tampon (1) à l'aide des sections de compression (9, 10), une forme de surface convexe de la zone comprimée du tampon (1) est obtenue.

9. Dispositif selon la revendication 8, **caractérisé en ce que** la direction d'insertion du tampon (1) dans la forme de compression (2) est essentiellement parallèle au plan de compression.

10. Dispositif selon l'une des revendications 8 ou 9, **caractérisé en ce que** chaque section de compression (5, 6, 9, 10) est formée selon la forme de la zone de compression à obtenir.

11. Dispositif selon l'une des revendications 8 à 10, **caractérisé en ce qu'**au moins une forme de compression (2, 8) peut être chauffée.

12. Dispositif selon l'une des revendications 8 à 11, **caractérisé en ce qu'**au moins une forme de compression (2, 8) comprend au moins deux mâchoires de compression (3, 4, 15, 16) en forme de plaques, les sections orientées l'une vers l'autre des arêtes frontales des mâchoires de compression (3, 4, 15, 16) étant conçues comme des sections de compression (5, 6, 9, 10).

13. Dispositif selon l'une des revendications 8 à 12, **caractérisé en ce qu'**il comprend au moins deux formes de compression (2, 8), qui sont conçus selon l'une des revendications 10 à 14, les sections de compression (9, 10) de la deuxième forme de compression (8) étant plus fortement incurvées que les sections de compression (5, 6) de la première forme de compression (2).
